# EUROPEAN PATENT APPLICATION

(11) **EP 3 545 849 A1**
(43) Date of publication of application: **02.10.2019**
(21) Application number: 18164256.2
(22) Date of filing: 27.03.2018
(51) Int. Cl.: A61B 8/08

(54) **APPARATUS, SYSTEM AND METHOD FOR VISUALIZING A PERIODICALLY MOVING ANATOMY**

(71) Applicant: Koninklijke Philips N.V., 5656 AE Eindhoven (NL)
(72) Inventor: BELT, Harm Jan Willem, 5656 AE Eindhoven (NL)
(74) Representative: de Haan, Poul Erik

(57) **Abstract**

The invention relates to an apparatus, a system and a method for visualizing measurement signals of a periodically moving anatomy. A processor receives temporal measurement signals comprising temporal information on the movement of a distal portion of an interventional device within the anatomy, and temporal information of the periodically moving anatomy. It is ascertained whether the temporal information on the movement of the distal portion of the interventional device within the anatomy are aperiodic or periodic. Based on whether the signals are ascertained aperiodic or periodic, the processor outputs to a display the temporal information of the periodically moving anatomy as received in real-time or as gated according to a predetermined phase of a measured or ascertained period.

## Description

### FIELD OF THE INVENTION

The invention relates to an apparatus, a system and a method for visualizing a periodically moving anatomy.

### BACKGROUND OF THE INVENTION

US 2015/0038842 discloses an imaging system for imaging a periodically moving object such as a heart for monitoring cardiac ablation procedures. An assigning unit assigns ultrasound signals like A-lines to motion phases based on a provided phase signal, wherein an ultrasound images generation unit generates several ultrasound images like gated M-mode images for the different motion phases based on the ultrasound signals assigned to the respective motion phase. A selecting unit is used to select an ultrasound image from the generated ultrasound images, wherein a display unit displays the selected ultrasound image. The selected ultrasound image corresponds therefore to a single motion phase such that motion artifacts in the displayed ultrasound image are reduced.

### SUMMARY OF THE INVENTION

It is an object of the present invention to provide an apparatus, a system and a method for improved visualization of a periodically moving anatomy.

In a first aspect of the invention an apparatus for visualizing measurement information of a periodically moving anatomy is provided, wherein the apparatus comprises a processor configured to:
receive temporal measurement signals comprising temporal information on the movement of a distal portion of an interventional device and temporal information of the periodically moving anatomy;
ascertain whether the temporal information on the movement of the distal portion of the interventional device is periodic or aperiodic;
gate the temporal information of the periodically moving anatomy when periodicity is ascertained;
output the temporal information of the periodically moving anatomy as received when the temporal information on the movement of the distal portion of the interventional device is ascertained aperiodic, and
output gated temporal information of the periodically moving anatomy when the temporal information on the movement of the distal portion of the interventional device is ascertained periodic.

The apparatus automatically outputs the temporal information of the periodically moving anatomy as received when the temporal information on the movement of the distal portion of the interventional device is aperiodic, and automatically switches to outputting gated temporal information of the periodically moving anatomy when the temporal information on the movement of the distal portion of the interventional device is periodic. The switching back from gated to as received temporal information of the periodically moving anatomy is also automatized.

A physician or assisting personal is interested in real-time anatomical information when navigating the interventional device to a target location in a clinical procedure, e.g. in a vessel of the heart. The temporal measurement information of the periodically moving heart are output as received, because the physician or assisting personal needs real-time feedback on their actions when maneuvering the interventional device to the target location, since quick reaction is required to correct the trajectory when the distal portion of the interventional device takes an undesired pathway. Once the target location is reached by the distal portion of the interventional device, the interest of the physician or assisting personal is to obtain stable measurements or images. The inventor came to the insight that when the distal portion of the interventional device is not advanced or retracted from a target position within the periodically moving anatomy, it follows at least partially the periodical movement of the anatomy, even when the distal portion of the interventional device is not in contact with the tissue of the periodically moving anatomy, e.g. heart. Additionally, when the interventional device is in contact with the tissue, due to the compliant nature of the tissue, the morphological information acquired by a sensor integrated on the distal portion of the interventional device comprises periodic pattern due to contraction and relaxation of the tissue during periodic movement of the anatomy. As result, temporal information on the movement of the distal portion of the interventional device can be extracted from the relative position of the sensor with respect to the tissue morphology. In consequence, the temporal information on the movement of the distal portion of the interventional device is an excellent indicator whether the user of the interventional device is in a navigation phase of the medical procedure toward a target location within the periodically moving anatomy, or whether the distal portion of the interventional device has reached the target location because the user stopped advancing or retracting the interventional device with respect to an entry point of the interventional device into the body. Accordingly, the temporal information on the movement of the distal portion of the interventional device can be used for automatically switching between real-time imaging or gated imaging, based on whether the temporal information on the movement of the distal portion of the interventional device is aperiodic or periodic.

In a second aspect of the invention a system for visualizing information of a periodically moving anatomy is provided, comprising: the apparatus according to the invention; the interventional device; and a display configured to display the information output by the processor. The system displays automatically temporal information of the periodically moving anatomy as received during navigation of the distal portion of the interventional device to the target location, and gated temporal information of the periodically moving anatomy when the distal portion of the interventional device is kept at the target location within the periodically moving anatomy. As result, the physician or assisting personal receives relevant information automatically, according to the relevant phase of the medical procedure. The interventional device may be a catheter, a guidewire, an interventional needle, a sheath, an electrical lead.

In an embodiment, the system further comprises an extracorporeal imaging unit configured to provide the temporal information on the movement of the distal portion of the interventional device and the temporal information of the periodically moving anatomy. The extracorporeal imaging unit may use one of the well-established imaging modalities in clinical procedures, e.g. ultrasound, radiological (X-ray) imaging including radiological angiography and computed tomography angiography (RA), magnetic resonance (MR) including magnetic resonance angiography (MRA), which have the capability to provide besides the morphological information of the anatomy also temporal information on the movement of the distal portion of the interventional device. Each of the imaging modalities has its own particular marker known in the art, which is detectible and visible in the respective imaging modality when the marker is integrated into the distal portion of the interventional device, e.g. echogenic markers for ultrasound imaging, electromagnetic marker for MR imaging, radiopaque markers for X-ray imaging. Accordingly, well-established extracorporeal imaging modalities are usable in the invention.

In another embodiment of the system, the extracorporeal imaging unit is configured to provide the temporal information of the periodically moving anatomy, and the distal portion of the interventional device comprises at least one sensor configured to provide the temporal information on the movement of the distal portion of the interventional device. The sensor may provide temporal information about its position, which may be acquired by using one of the tracking techniques known in the art: electromagnetic, complex electrical impedance, optical shape sensing, ultrasound tracking, global positioning sensing.

In an alternative embodiment of the system, the distal portion of the interventional device comprises at least one sensor configured to provide the temporal information on the movement of the distal portion of the interventional device and the temporal information of the periodically moving anatomy. The sensor may be an ultrasound transducer or an optical coherence tomography (OCT) sensor. The system automatically switches between displaying temporal information of the periodically moving anatomy in real-time as received and gated temporal information of the periodically moving anatomy, based on whether the user is in a navigation phase of the medical procedure or whether the user reached a target location within the anatomical structure, without the need of an extracorporeal imaging unit. The morphological information of the anatomy acquired by the sensor integrated on the distal portion of the interventional device comprises periodic pattern due to contraction and relaxation of the tissue during periodic movement of the anatomy. Thus, the temporal information on the movement of the distal portion of the interventional device can be extracted from the relative position of the sensor with respect to the tissue morphology.

In an embodiment of the system, the temporal information on the movement of the distal portion of the interventional device and the temporal information of the periodically moving anatomy are ultrasound signals, and the temporal information on the movement of the distal portion of the interventional device is based on the movement of the sensor relative to the anatomy. Due to the compliant nature of the tissue, when the interventional device is in contact with the tissue, the morphological information acquired by a sensor comprises periodic pattern due to contraction and relaxation of the tissue during periodic movement of the anatomy. As result, temporal information on the movement of a distal portion of an interventional device can be extracted from the relative position of the sensor with respect to the morphology of the tissue, e.g. vessel wall, heart chamber wall, septum. The use of ionizing radiation due to X-ray based imaging is avoided in the clinical procedure and no bolus of contrast agent is needed to be used. Since the anatomy is imaged in real-time with ultrasound and an anatomical map can be created on the run, potential change of the anatomy (e.g. deformation, displacement) between the screening or planning phase and the intervention is avoided. Furthermore, the ultrasound map created real-time can be used to immediately detect any potential change of the anatomy between a pre-procedural anatomical map from screening/planning and the real-time medical procedure.

In an embodiment of the system, the at least one sensor is configured to provide OCT signals, and the temporal information on the movement of the distal portion of the interventional device is based on the movement of the sensor relative to the anatomy. Similar to the previous embodiment, temporal information on the movement of the distal portion of the interventional device can be extracted from the relative position of the sensor with respect to the morphology of the tissue.

In an embodiment of the system, the processor is configured to render the temporal information of the periodically moving anatomy into OCT or ultrasound images of the anatomy, according to at least one of a motion-mode (M-mode), a two-dimensional (2D), a three-dimensional (3D) and a cross-plane visualization modality.

In a further embodiment of the system, the periodicity is ascertained based on a temporal value-string signal obtained by deriving an average or a median value from at least a portion of the images in real-time.

In an embodiment, the system further comprises a unit configured to provide to the processor measurements of temporal extracorporeal electrocardiogram signals of the periodically moving anatomy, wherein the processor is configured to gate the temporal information of the periodically moving anatomy according to a phase of a period of the measured temporal extracorporeal electrocardiogram signals. It is general practice to use an electrocardiograph for measuring electrocardiogram during medical diagnostic or treatment procedures. Once the temporal information on the movement of the distal portion of the interventional device is ascertained periodic, in other words when periodicity is ascertained, the gating of the temporal information of the periodically moving anatomy can be triggered based on a predetermined phase of the period of the measured temporal extracorporeal electrocardiogram signals. Therefore, the received temporal information on the movement of the distal portion of the interventional device is only used for ascertaining whether it is periodic or aperiodic, whereas the electrocardiogram signals are used for gating, which allows the physician to select any phase of the period not only during the procedure, but even before the medical procedure starts.

In an embodiment of the system, the at least one sensor is configured to provide to the processor temporal spatial location measurement signals as temporal information on the movement of the distal portion of the interventional device, based on which the processor is configured to ascertain the periodicity. The sensor may be one of an electromagnetic tracking sensor, a magnetic field sensor, an electrical sensor for impedance based tracking, optical shape sensor, GPS sensor.

In an embodiment of the system, the at least one sensor is configured to provide to the processor temporal electrical measurement signals of the periodically moving anatomy, resulting from an electrical activation of the anatomy, based on which the processor is configured to gate the temporal information of the periodically moving anatomy. The periodic movement of the anatomy may be intrinsic but also a result of electrical pacing, and since the pacing signal can be detected by the at least one electrical sensor, it can be used for gating of the temporal information of the periodically moving anatomy.

In any of the embodiments, the system may further comprise a user interface configured for selecting a phase of the period according to which the temporal information of the periodically moving anatomy is gated. A physician or assisting personal may select the phase by means of a user interface such as a pointing device coupled to the apparatus or by means of a display comprising touchscreen. Different phases may be selected during a procedure, according to the phase of interest for particular medical procedures. For example the physician may be interested to select different phases to assess the condition of the cardiac valve during diagnosis, to decide on valve deployment and to assess the success of the cardiac valve replacement. Another example is when a coronary vessel is diagnosed, a stent is deployed and the stent placement is assessed.

In a further embodiment of the system, the at least one sensor is further configured to provide intracorporeal temporal information of the periodically moving anatomy having higher resolution and smaller field of depth than the temporal information of the periodically moving anatomy provided by the extracorporeal imaging unit, wherein the processor is further configured to:
register the extracorporeal and intracorporeal information of the periodically moving anatomy,
output to the display the extracorporeal temporal information of the periodically moving anatomy augmented by the intracorporeal information of the periodically moving anatomy. The display may visualize intracorporeal images overlaid onto extracorporeal images at a position corresponding to the location of the sensor on the distal portion of the interventional device in the extracorporeal image. In an alternative embodiment, the processor can be configured to compute the field of depth of the temporal intracorporeal information of the periodically moving anatomy and to render on the display extracorporeal images wherein the information according to the computed field of depth is substituted by the intracorporeal images. This alternative has particularly benefit where both the extracorporeal and intracorporeal images are originating from the same imaging modality, in particular where ultrasound information is acquired at different ultrasound frequencies by the ultrasound transducer on the interventional device and the extracorporeal imaging probe.

In a third aspect of the invention a method of visualizing measurement signals of a periodically moving anatomy is provided, comprising the steps: receiving temporal measurement signals by a processor, comprising temporal information on the movement of a distal portion of an interventional device and temporal information of the periodically moving anatomy;
ascertaining by the processor whether the temporal information on the movement of the distal portion of the interventional device is periodic or aperiodic;
gating the temporal information of the periodically moving anatomy when periodicity is ascertained;
outputting the temporal information of the periodically moving anatomy as received when the temporal information on the movement of the distal portion of the interventional device is ascertained aperiodic, and
outputting gated temporal information of the periodically moving anatomy when the temporal information on the movement of the distal portion of the interventional device is ascertained periodic.

The method creates optimal visualization for a physician or assisting personal during medical procedures comprising navigation of the distal portion of the interventional device toward a target location under real-time imaging of the anatomy, and diagnostic or treatment of the target location under gated imaging. The switching from gated to as received temporal information of the periodically moving anatomy is done automatically. In a particular example, the distal portion of a catheter is introduced into the inferior vena cava through the groin and advanced toward the heart. During navigation, the distal portion of the interventional device does not follow the periodical movement pattern of the heart, and the temporal information on the movement of a distal portion of the catheter is aperiodic, which means that the navigation phase occurs under real-time imaging. That is still the case when the distal portion of the catheter is in the heart and a target position or site is searched by further moving the distal portion of the catheter within the heart. Once the physician is content that the distal portion of the catheter reached the location where diagnostic measurement (e.g. intracardiac echography) or treatment of the heart (e.g. release of therapeutic agent) is desired, the portion of the catheter proximal to the site of introduction of the catheter into the body is fixed with respect to the site of introduction. Under these circumstances the distal portion of the catheter partially follows the periodical movement of the heart tissue even when the distal portion of the interventional device is not in contact with the tissue. Accordingly, the temporal information on the movement of the distal portion of the catheter can be used for automatically switching between real-time imaging and gated imaging, based on whether the temporal information on the movement of the distal portion of the catheter is aperiodic or periodic.

Additionally, due to the compliant nature of the tissue, when the distal portion of the catheter is in contact with the cardiac tissue, the morphological information acquired by a sensor integrated on the distal portion of the catheter comprises periodic pattern due to contraction and relaxation of the cardiac tissue during periodic movement of the anatomy. Therefore, the temporal information on the movement of the distal portion of the catheter can also be extracted from the relative position of the sensor with respect to the morphology of the cardiac tissue.

When during the navigation phase toward the target location the user stops advancing or retracting the interventional device, and the temporal information on the movement of the distal portion of the interventional device becomes periodic, according to the method gated temporal information of the periodically moving anatomy will be output. The physician or assisting personal can assess in stable visualization conditions whether the target location is reached or whether the distal portion of the interventional device needs to be further advanced or retracted.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of the system for visualization of a periodically moving anatomy, according to the invention.
Fig. 2 shows schematically and exemplarily an embodiment of the interventional device used for providing temporal intracorporeal measurement signals.
Fig. 3 shows a flowchart exemplarily illustrating an embodiment of a visualizing method of measurement signals of a periodically moving anatomy.
Figs. 4-7 show exemplary temporal value-string signals obtained.
Fig. 8 is a schematic illustration of the gating process of the temporal information of the periodically moving anatomy.
Fig. 9 is a schematic illustration of an embodiment of the gating step for the method.
Figs. 10,11 show exemplary temporal extracorporeal electrocardiogram signals used for the gating step of the method.
Fig. 12 is an exemplary 3D ultrasound image of the heart chamber in the presence of the distal portion of the interventional device.

### DETAILED DESCRIPTION OF EMBODIMENTS

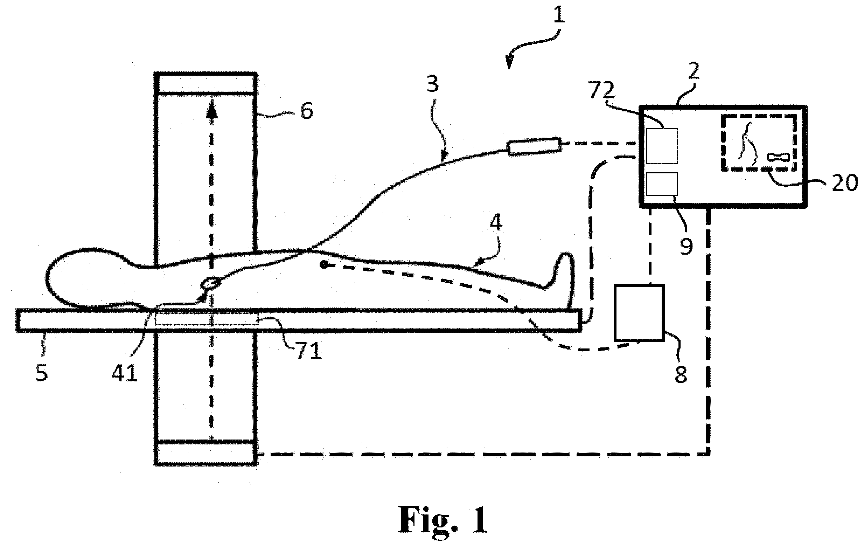

In Fig. 1 an embodiment of the system 1 for characterization and visualization of an anatomical structure of a living being 4 is presented, wherein the living being may be a person or an animal. The system 1 comprises an apparatus 2 and an interventional device 3, wherein the interventional device is configured to provide temporal measurement signals from within an anatomical structure of the patient 4, e.g. a heart 41 or a vessel of the circulatory system. The apparatus 2 comprises a processor 9 configured to process the temporal measurement signals, to ascertain characteristics of the anatomical structure, and to output data based on the processed signals to a display. The apparatus 2 may comprise an integrated display 20 for displaying images of the anatomical structure rendered based on the processed signals. Alternatively or additionally, the apparatus may be configured to provide the processed signals to an external display.

In an embodiment, the system may comprise an extracorporeal imaging unit 6 for acquiring 2D or 3D morphological information of the anatomical structure of interest and/or the location of the interventional device within the anatomical structure either upon injection of a contrast agent bolus into the target anatomical structure or without the use of contrast agent. The injection of the contrast agent bolus into the target vasculature is performed either with the interventional device 3 or with a different medical instrument. 2D or 3D morphology of the anatomy can be obtained by various well established techniques such as: ultrasound, X-ray including RA, MR including MRA. For the respective imaging modalities contrast agents are available, for instance radiological contrast agent for RA, a gadolinium-based substance for MRA, echogenic contrast agent comprising microbubbles for extracorporeal or intracorporeal ultrasound imaging.

The system may further comprise a tracking unit 72 for receiving temporal measurement information about the location of a distal portion of the interventional device 3 within the anatomical structure of the patient 4. Spatial position tracking of the location of the device may be based on one of an electromagnetic, an optical, an ultrasound, an electrical impedance, global position sensing or a radiological principle. Electromagnetic tracking of interventional devices is based on detection of an external electromagnetic field by electromagnetic sensors integrated into the distal portion of the interventional device 3. The electromagnetic field generator 71 may be fixedly of removably attached to either the support means 5 of the patient 4 or to the imaging unit 6. Multiple electromagnetic sensors integrated into the interventional device 3 allow simultaneous localization of multiple points along the device 3, based on which the shape of a segment of at least the distal portion of the device 3 can be reconstructed by connecting the localized points.

In an alternative embodiment, the tracking of the temporal location of the instrument is derived from laser radiation. For optical tracking, the interventional device comprises multiple optical fibers or a multi-core optical fiber extending from a distal portion to a proximal portion of the interventional device 3, typically the handgrip, where optical connection to the tracking unit 72 is facilitated. The tracking unit 72 comprises a laser radiation generator configured to transmit laser radiation into the at least one optical fiber integrated within the interventional device, and an optical detector configured to receive reflections from within the at least one optical fiber. The shape of the at least one optical fiber, hence that of the device 3 is derived by processing optical reflection signals from within the optical fibers, received by a detector. The reflections of the laser radiation from within the at least one optical fiber are indicative of local strains to which the device 3 is exposed in tortuous vessel branches. Shape determination of a medical instrument based on optical shape sensing is described in more details in WO 2008131303. Optical shape sensing makes possible the localization of any points along the at least one optical fiber integrated into the interventional device 3, for all instances.

In an alternative embodiment, the location tracking technique of the instrument is based on electrical impedance measurement. Electrical signals are sent into the patient by multiple electrically conductive patches distributed onto the body of the patient 4. The distal portion of the trackable interventional device 3 comprises spatially distributed electrically conductive electrodes. The electrical impedances derived from the received electrical signals are indicative of the locations of the conductive electrodes in the region of interest comprising the target anatomy.

In yet a further alternative embodiment, the location of the distal portion of the interventional device 3 is tracked by using the imaging unit. The interventional device comprises specific sensors sensitive to the source of the imaging unit used in the procedure. For extracorporeal ultrasound imaging the distal portion of the interventional device 3 comprises an ultrasound sensor or multiple ultrasound sensors distributed spatially. Upon excitation of the external ultrasound imaging unit with an electric signal, the generated ultrasound signal is transmitted into the body of the patient 4 and a fraction of the ultrasound signal is received by the ultrasound sensors integrated into the interventional device 3. The time of flight of the ultrasound signals is indicative of the locations of the ultrasound sensors integrated into the device 3 with respect to the external ultrasound imaging unit, and by processing the signals the location and/or shape of the distal portion of the interventional device comprising the ultrasound sensors can be reconstructed. In an alternative embodiment, the ultrasound signals are sent by the ultrasound sensors integrated into the device 3 and are received by the external ultrasound unit. The external ultrasound imaging unit may be a system with an ultrasound probe suitable for transthoracic echography (TTE), whereas the interventional device may be one of a an intracardiac echography (ICE) catheter, a transesophageal echocardiography device (TEE), a guidewire or an ablation catheter comprising ultrasound sensor.

In yet a further alternative, the location of the distal portion of the device 3 may be derived from the measurement signals provided by the external imaging unit. In particular, when the external imaging unit uses X-ray imaging and the distal portion of the interventional device comprises radiopaque marker.

The system may further comprise an electrical signal measurement unit 8, e.g. an electrocardiograph, configured to provide to the processor measurements of temporal extracorporeal electrocardiogram signals of the periodically moving anatomical structure, which are measured on the surface of the body of the patient.

The system may further comprise user interface through which various parameters are adjustable or preferences are selectable by the physician and/or assisting personal during the medical diagnostic or treatment process.

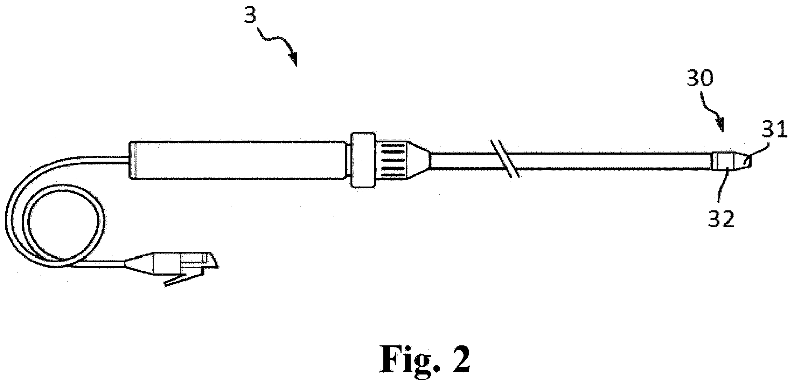

Fig. 2 shows schematically and exemplarily an embodiment of the interventional device 3 usable for providing temporal intracorporeal measurement signals. The interventional device may be one of a guiding catheter, a sheath, a diagnostic catheter, a guidewire, a treatment catheter, an interventional needle. The interventional device may be steerable for navigation, the proximal portion of the interventional device comprising the means for controlling a bending of the distal portion, such as a turning wheel or a slider. The distal portion 30 of the interventional device 3 comprises a sensor 31 configured for acquiring temporal intracorporeal measurement signals of the periodically moving anatomical structure, which are transmitted to the processor 9. The sensor may be one of an ultrasound transducer or an OCT sensor. The distal portion of the interventional device may additionally comprise a sensor 32 configured for acquiring temporal measurement signals on the location of the distal portion of the interventional device within the anatomical structure of the patient and which are transmitted to the processor of the apparatus. The sensor 32 may be one of the sensors listed in the description of the location tracking techniques, referring to the system illustrated in Fig. 1.

Alternatively, the sensor 32 may be an electrical sensor configured to provide to the processor temporal electrical measurement signals of the periodically moving anatomical structure upon an electrical activation, e.g. intrinsic electrical activation signals of the heart or electrical activation signals resulting from pacing signals provided to the heart by an additional electrode.

The transmission of the temporal intracorporeal measurement signals between the sensors 31,32 and the processor may be realized by wired or wireless connection.

In some embodiments, one sensor 31 may have both functionalities, acquiring temporal measurement signals on the location of the distal portion of the interventional device within the anatomy and acquiring temporal intracorporeal measurement signals of the periodically moving anatomy. In one example, an ultrasound sensor may provide temporal ultrasound information of the morphology of the anatomy, it may measure blood flow, blood pressure, it may provide location information of the ultrasound sensor with respect to an external ultrasound probe, it may provide temporal information on the movement of the ultrasound sensor relative to an anatomical structure. Such an ultrasound senor can be a capacitive micromachined ultrasonic transducer (CMUT). In a second example, an electrical sensor may be used to provide location information of the electrical sensor with respect to an external electrical sensor, to measure electrical activation signal of a heart either intrinsic or induced by pacing, it may measure blood pressure. In a third example, an optical sensor may provide temporal optical coherence tomography signals of the anatomical structure, it may measure blood flow, blood pressure, it may provide temporal information on the movement of the optical sensor relative to the anatomical structure.

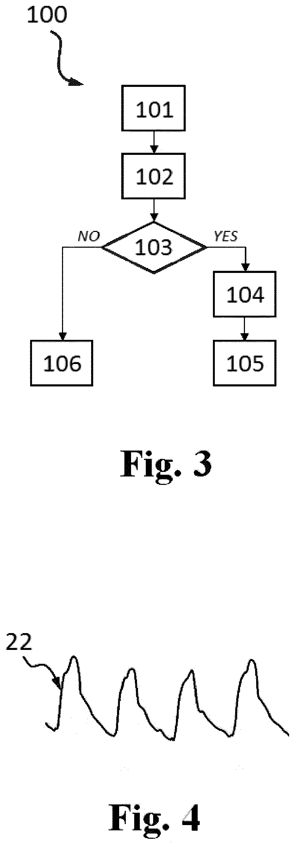

Fig. 3 shows a flowchart exemplarily illustrating an embodiment of a visualizing method 100 of measurement signals of a periodically moving anatomy. In step 101 the processor 9 receives temporal measurement signals comprising temporal information on the movement of the distal portion 30 of the interventional device 3 and temporal information of the periodically moving anatomy 41.

In a first embodiment, an extracorporeal imaging unit 6 is configured to provide both, the temporal information on the movement of the distal portion 30 of the interventional device 3 and the temporal information of the periodically moving anatomy 41. The extracorporeal imaging unit may use ultrasound imaging, X-ray imaging or MR imaging, in conjunction with a region of the distal portion of the interventional device configured to be visible in the respective imaging modality. Accordingly, the extracorporeal imaging unit provides temporal measurement signals of the anatomy, e.g. morphological information. Additionally, the temporal information on the movement of a distal portion 30 of an interventional device 3 can be derived from the temporal measurement signals due to the visibility of the region of the distal portion of the interventional device.

In a second embodiment, an extracorporeal imaging unit 6 is configured to provide the temporal information of the periodically moving anatomy 41, and the distal portion 30 of the interventional device 3 comprises at least one sensor 31,32 configured to provide the temporal information on the movement of the distal portion 30 of the interventional device 3. The extracorporeal imaging unit may provide the temporal information of the periodically moving anatomy in any of the imaging modalities listed for the first embodiment.

In a third embodiment, the distal portion 30 of the interventional device 3 comprises at least one sensor 31,32 configured to provide the temporal information on the movement of the distal portion 30 of the interventional device 3 and the temporal information of the periodically moving anatomy.

The at least one sensor 31,32 in the second and third embodiments may be one of the sensors described in the section referring to Fig. 2, which may be used standalone or separately to provide simultaneously the temporal information on the movement of the distal portion 30 of the interventional device 3 and the temporal information of the periodically moving anatomy.

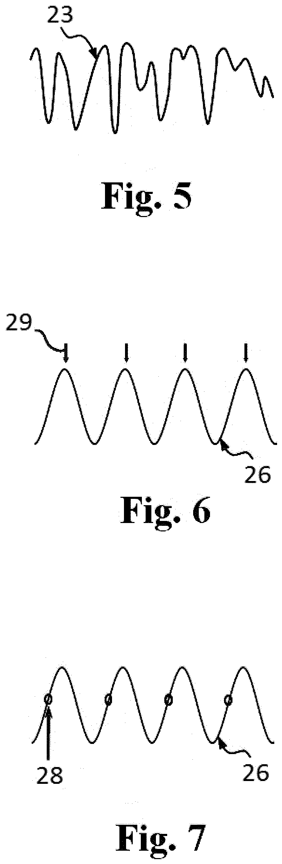

In step 102 the processor 9 processes the temporal information on the movement of the distal portion 30 of the interventional device 3 relative to the anatomy. In one of the alternatives, relevant to the first and second embodiments, a value-string along a temporal axis is obtained from temporal information on the movement of the distal portion 30 of the interventional device 3, which is derived from the location information of the distal portion of the interventional device acquired by the extracorporeal imaging unit, or from the location information of the sensor integrated into the distal portion of the interventional device with respect to the extracorporeal imaging unit. In a different alternative, relevant to the second and the third embodiments, the temporal information on the movement of the distal portion 30 of the interventional device 3 relative to the anatomy is obtained from the temporal measurement signals acquired by the sensor and used for rendering images of the anatomy according to at least one of an M-mode, a 2D, a 3D and a cross-plane visualization modality. A temporal value-string signal either periodic 22 or aperiodic 23, illustrated in Figs. 4 and 5, is obtained by deriving an average or a median value from at least a portion of the images in real-time. For example, the temporal value-string signal is derived from one of the 2D, cross-plane or 3D ultrasound image sequence by taking the mean value of all pixels (for 2D, cross-plane) or voxels (3D) in each image from the original image sequence, or by taking the median value of all pixels (2D, cross-plane) or voxels (3D) in each image from the original image sequence, or taking another single statistical parameter from all pixels (2D, cross-plane) or voxels (3D) in each image from the original image sequence. In another alternative, wherein the sensor provides images (e.g. ultrasound or OCT), the temporal value-string signal is derived from the distance of the sensor to an anatomical structure in a predefined direction or in a predefined region. In Figs. 6,7 such a value-string signal 26 is exemplarily shown, wherein the value-string signal is offset by the average of the distance for a time interval. For instance, the distance of the vessel wall to the sensor can be derived from an M-mode image, since the M-mode image is composed by information acquired along a single line in a certain time interval. Similarly, from 2D or 3D images the distance to the sensor can be derived as an average or a median value along the plurality of lines for the various angles to the vessel wall of the region of interest. Furthermore, the temporal 2D or 3D images are composed of a plurality of M-mode images, from which one or several can be selected to derive the temporal value-string signal.

Alternatively, the temporal value-string signal may be a binary signal derived from the temporal M-mode image comprising periodic pattern due to contraction and relaxation of the cardiac tissue during periodic movement of the heart, e.g. according to the description from the 3^{rd} paragraph of column 12 to the antepenultimate paragraph of column 14, referring to Figs. 5A-5C and Fig. 6, in US 8,774,906.

The temporal value-string signal may be derived from ultrasound M-mode image, as described in US 8,774,906, or it may be derived from OCT M-mode image as presented in Fig. 3A on page 3802 of the article by Airong Li et al. in Human Molecular Genetics, 2013, Vol. 22, No. 18, pages 3798-3806 (doi:10.1093/hmg/ddt230). The temporal value-string signal may alternatively be derived directly from the raw or processed ultrasound or OCT signals, before the M-mode image is rendered.

In step 103 the processor ascertains whether the temporal information on the movement of the distal portion 30 of the interventional device 3 is periodic or aperiodic. In an example, it is ascertained whether the temporal value-string signal 22,23 from step 102 is periodic or aperiodic by digital spectral analysis. Based on the short-time Fourier spectrum the running temporal value-string signal is windowed (in time) followed by the computation of the spectrum using the absolute values of the complex fast Fourier transformation (FFT) result. Subsequently, from the short-time Fourier spectrum it is computed at each time the bandwidth and the center frequency. When the bandwidth is sufficiently small (below a fixed threshold value) then the decision is taken that the signal is periodic, while the signal is aperiodic otherwise. When the signal is ascertained periodic, the period of the cycle is computed from the inverse value of the center frequency. Although it is faster to ascertain periodicity of a temporal value-string signal 22,23 because it requires less computation power of the processor, in an alternative of any of the three embodiments disclosed in step 101 the periodicity of the temporal information on the movement of the distal portion 30 of the interventional device 3 can be ascertained directly from the temporal measurement signal as received for the respective embodiments. In the first and second embodiments the location information of the distal portion of the interventional device acquired by the extracorporeal imaging unit or by the sensor integrated into the distal portion of the interventional device with respect to the extracorporeal imaging unit may be directly used for ascertaining periodicity, whether the location information is related to 2D or 3D space. In a further alternative, relevant for the second and third embodiments, the distance of the anatomical structure to the sensor may be a 2D or 3D matrix derived from the respective 2D or 3D images and representing the coordinates of the anatomical structure or vectors defined by the magnitude and direction of the anatomical structure relative to the sensor in the respective 2D or 3D space.

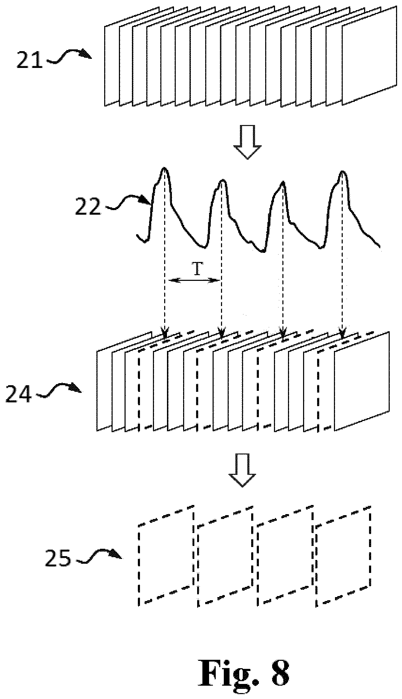

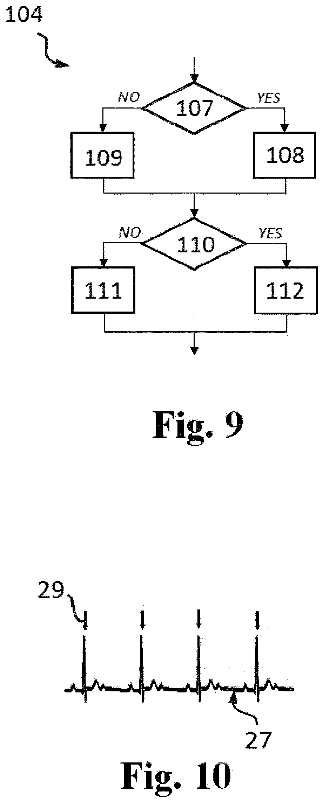

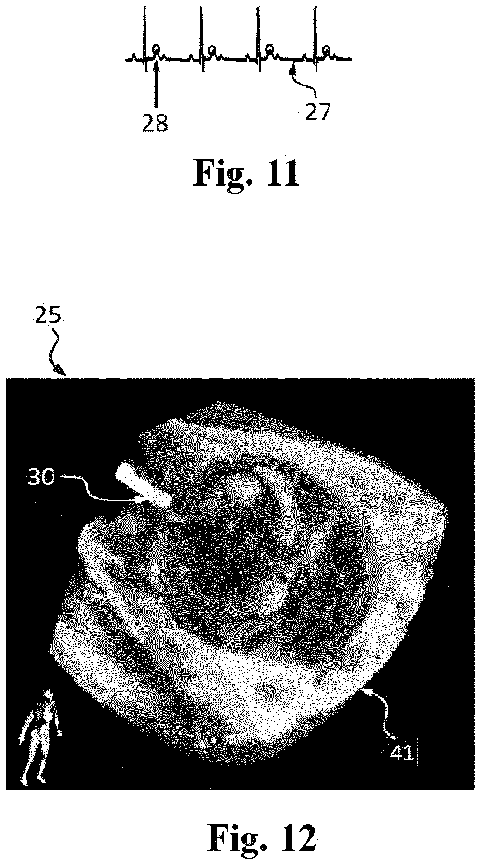

In the case that it is ascertained that the temporal information on the movement of the distal portion 30 of the interventional device 3 is periodic, such as illustrated with 22 in Fig. 8, the processor triggers gating 24 of the temporal information of the periodically moving anatomy 41 in step 104. In other words, when periodicity is ascertained in step 103, the method proceeds to the gating step 104. In Fig. 9 the gating step 104 is schematically illustrated, comprising steps which may optionally be missing. In step 107 the options branch out in two possibilities, depending on the predetermined configuration of the system, which can be adjusted by the physician or assisting personal via the user interface. When in step 107 the choice is detected that the gating should be based on the temporal information on the movement of the distal portion 30 of the interventional device 3 directly or based on the temporal value-string signal 22 derived from it in step 102, then in step 108 the gating will be performed based on that particular signal. On the other hand, if in step 107 the choice is detected that the gating should be based on a different signal than the temporal information on the movement of the distal portion 30 of the interventional device 3, then the signal for gating can be selected by the user in step 109. The gating signal may be one of a temporal extracorporeal electrocardiogram signal 27 (Figs. 10,11) provided by the electrical signal measurement unit 8, e.g. electrocardiograph, or temporal intracorporeal electrogram signals provided by the at least one sensor integrated on the distal portion of the interventional device.

The phase of the periodic signal used for gating may be predetermined in the configuration of the system and/or adjusted by the user through the user interface. When in step 110 it is determined that the phase of the period T should be selected 28 by the user (Figs. 7,11), then in step 111 the user interface will request from the user to introduce or confirm a selected phase. In other instances the processor uses automatic peak detection 29 (Figs. 6,10) in step 112, or uses a certain predetermined phase that is delayed from the automatically detected peaks 29. Once the phase of the period T is selected either by the user or is automatically detected by the processor, the gating 24 of the temporal information of the periodically moving anatomy 41 is accomplished, resulting in gated temporal information 25 of the periodically moving anatomy 41 (Fig. 8). The gated temporal information 25 of the periodically moving anatomy 41 can be a sequence of image frames corresponding to the same phase of consecutive periods used for gating, or other temporal measurement data of the periodically moving anatomy 41 corresponding to the same phase of consecutive periods used for gating.

In step 105 the processor outputs gated temporal information of the periodically moving anatomy 41 when the temporal information on the movement of the distal portion 30 of the interventional device 3 is ascertained periodic, and in step 106 outputs the temporal information of the periodically moving anatomy 41 as received when the temporal information on the movement of the distal portion 30 of the interventional device 3 is ascertained aperiodic. As result, in any of the first to the third embodiment or their further alternative variations the processor outputs gated temporal information of the periodically moving anatomy or temporal information of the periodically moving anatomy as received at any instances during operation of the system. The gated temporal information of the periodically moving anatomy may be one of the M-mode, 2D, cross-plane, 3D visualization modalities of any of the ultrasound, X-ray, OCT, MR imaging modalities.

In a clinical example the anatomy is a heart. For the navigation of an interventional device to a target location within a chamber of the heart or a vessel of the heart it is favorable that the temporal measurement signals of the periodically moving heart are output as received, due to the fact that the physician or assisting personal needs real-time feedback to find the target location and it has to quickly react and correct the trajectory when the distal portion of the interventional device takes an undesired pathway. During navigation the distal portion of the interventional device is advanced or retracted with respect to the target location of interest. On the other hand, when the target location is reached by the distal portion of the interventional device, the interest of the physician or assisting personal is to obtain stable measurements or images. Since the measurements are influenced by the periodic movement of the heart, real-time outputting of the temporal measurement signal causes rapid periodical change of the measurement signal, which may confuse the physician or assisting personal in taking decision whether the circumstances are appropriate for acquiring diagnostic measurements or for performing a treatment procedure. According to any of the embodiments, the temporal information of the periodically moving heart will be gated automatically, the apparatus outputting only the gated information when the catheter is not advanced or retracted relative to a the target location. When the distal portion of the device is moved from the target location, the apparatus automatically switches to outputting the temporal information of the periodically moving heart as received, thereby automatically terminating outputting gated temporal information of the periodically moving heart. An exemplary visualization is illustrated on Fig. 12, wherein the 3D ultrasound image of the heart chamber 41 is shown in the presence of the distal portion 30 of the interventional device.

The images may further be post-processed by applying spatial and/or temporal filtering and adjusting the gray levels. The post-processing may require additional computation power, which may influence the real-time performance of the imaging.

In a further alternative of the second embodiment, the at least one sensor, e.g. sensor 31 in Fig. 2, is further configured to provide intracorporeal temporal information of the periodically moving anatomy 41 having higher resolution and smaller field of depth than the temporal information of the periodically moving anatomy 41 provided by the extracorporeal imaging unit 6. In a particular example the anatomy is heart, the intracorporeal temporal information of the heart is ultrasound information acquired by the ultrasound transducer 31 at a frequency in the interval of 20-45 MHz and the extracorporeal temporal information of the heart is ultrasound information acquired by an extracorporeal ultrasound probe at a frequency in the interval of 1-10 MHz. In other alternatives the extracorporeal temporal information of a vessel of the heart is ultrasound information and the intracorporeal temporal information of the vessel of the heart is OCT information. In a further alternative the extracorporeal temporal information of the heart is X-ray information and the intracorporeal temporal information of the vessel of the heart is OCT or ultrasound information. The processor is further configured to register the extracorporeal and intracorporeal information of the periodically moving anatomy 41. The registration may be based on tracking of the sensor integrated on the distal portion of the interventional device by the extracorporeal imaging unit, by other tracking techniques listed in the description referring to the system illustrated in Fig. 1, or by automatic segmentation and identification of identical morphological features in both the intracorporeal and extracorporeal information of the periodically moving heart. In an alternative, the registration of the extracorporeal and intracorporeal information of the periodically moving anatomy is performed before the gating step, and the temporal intracorporeal information of the periodically moving anatomy is gated similarly as the temporal extracorporeal information of the periodically moving anatomy. The processor is further configured to output to the display 20 the extracorporeal temporal information of the periodically moving anatomy 41 augmented by the intracorporeal information of the periodically moving anatomy 41. In an alternative, the processor is configured to render on the display intracorporeal images overlaid onto extracorporeal images at a position corresponding to the location of the sensor on the distal portion of the interventional device in the extracorporeal image. In a different alternative, the processor is configured to compute the field of depth of the temporal intracorporeal information of the periodically moving anatomy and to render on the display extracorporeal images wherein the information according to the computed field of depth is substituted by the intracorporeal images. This alternative has particularly benefit where both the extracorporeal and intracorporeal images are ultrasound information acquired at different ultrasound frequencies.

Although in the detailed description of the invention and the examples the temporal information of the periodically moving anatomy is related to morphological information of the anatomy, the invention is applicable when the temporal information of the periodically moving anatomy comprises temporal physiological measurements such as blood pressure and blood flow measurements within the anatomy, that are measurable by one of the sensors 31 and 32, e.g. in vessel or in a chamber of the heart. Accordingly, in the respective embodiments of the system wherein at least one of the sensors 31,32 are integrated on the distal portion of the interventional device, the gated temporal physiological measurements can be output to the display additionally or alternatively to the gated temporal morphological information.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be provided, comprising program code means for causing apparatus 2 to carry out the steps of method 100 when the computer program is run on a computer controlling the apparatus. The computer program may be stored and/or distributed on a suitable medium, such as an optical storage medium or a solid-state medium, supplied together with or as part of other hardware, but may also be distributed in other forms, such as via the internet or other wired or wireless telecommunication systems.

Any reference signs in the claims should not be construed as limiting the scope.

## Claims

1. Apparatus (2) for visualizing measurement information of a periodically moving anatomy (41), wherein the apparatus comprises a processor (9) configured to:
receive temporal measurement signals (21) comprising temporal information on the movement of a distal portion (30) of an interventional device (3) and temporal information of the periodically moving anatomy (41);
ascertain whether the temporal information on the movement of the distal portion (30) of the interventional device (3) is periodic or aperiodic;
gate (24) the temporal information of the periodically moving anatomy (41) when periodicity is ascertained;
output the temporal information of the periodically moving anatomy (41) as received when the temporal information on the movement of the distal portion (30) of the interventional device (3) is ascertained aperiodic, and
output gated temporal information (25) of the periodically moving anatomy (41) when the temporal information on the movement of the distal portion (30) of the interventional device (3) is ascertained periodic.

2. A system (1) for visualizing information of a periodically moving anatomy (41), comprising:
an apparatus (2) according to claim 1;
the interventional device (3); and
a display (20) configured to display the information output by the processor.

3. The system of claim 2, further comprising an extracorporeal imaging unit (6) configured to provide the temporal information on the movement of the distal portion (30) of the interventional device (3) and the temporal information of the periodically moving anatomy (41).

4. The system of claim 2, further comprising an extracorporeal imaging unit (6) configured to provide the temporal information of the periodically moving anatomy (41), and wherein the distal portion (30) of the interventional device (3) comprises at least one sensor (31,32) configured to provide the temporal information on the movement of the distal portion (30) of the interventional device (3).

5. The system of claim 2, wherein the distal portion (30) of the interventional device (3) comprises at least one sensor (31,32) configured to provide the temporal information on the movement of the distal portion (30) of the interventional device (3) and the temporal information of the periodically moving anatomy (41).

6. The system of claim 4 or 5, wherein the temporal information on the movement of the distal portion (30) of the interventional device (3) and the temporal information of the periodically moving anatomy (41) are ultrasound signals, and wherein the temporal information on the movement of the distal portion (30) of the interventional device (3) is based on a movement of the at least one sensor relative to the anatomy (41).

7. The system of claim 4 or 5, wherein the at least one sensor is configured to provide optical coherence tomography signals, and wherein the temporal information on the movement of the distal portion (30) of the interventional device (3) is based on a movement of the at least one sensor relative to the anatomy (41).

8. The system of claim 6 or 7, wherein the processor is configured to render the temporal information of the periodically moving anatomy (41) into images of the anatomy according to at least one of an M-mode, a 2D, a 3D and a cross-plane visualization modality.

9. The system of claim 8, wherein the periodicity is ascertained based on a temporal value-string signal (22,23) obtained by deriving an average or a median value from at least a portion of the images in real-time.

10. The system of any of the claims 2 to 9, further comprising a unit (8) configured to provide to the processor measurements of temporal extracorporeal electrocardiogram signals (27) of the periodically moving anatomy, wherein the processor is configured to gate (24) the temporal information of the periodically moving anatomy (41) according to a phase of a period (T) of the measured temporal extracorporeal electrocardiogram signals.

11. The system of claim 4 or 5, wherein the at least one sensor is configured to provide to the processor temporal spatial location measurement signals (26) as temporal information on the movement of the distal portion (30) of the interventional device (3), upon which the processor is configured to ascertain the periodicity.

12. The system of claim 4 or 5, wherein the at least one sensor is configured to provide to the processor temporal electrical measurement signals of the periodically moving anatomy resulting from an electrical activation of the anatomy, upon which the processor is configured to gate the temporal information of the periodically moving anatomy (41).

13. The system according to any of the claims 2 to 12, further comprising a user interface configured for selecting a phase (28) of the period according to which the temporal information of the periodically moving anatomy (41) is gated.

14. The system of claim 4, wherein:
the at least one sensor is further configured to provide intracorporeal temporal information of the periodically moving anatomy (41) having higher resolution and smaller field of depth than the temporal information of the periodically moving anatomy (41) provided by the extracorporeal imaging unit,
wherein the processor is further configured to:
register the extracorporeal and intracorporeal information of the periodically moving anatomy (41),
output to the display the extracorporeal temporal information of the periodically moving anatomy (41) augmented by the intracorporeal information of the periodically moving anatomy (41).

15. A method (100) of visualizing measurement signals of a periodically moving anatomy (41), comprising the steps:
receiving (101) temporal measurement signals (26,21) by a processor (9), comprising temporal information on the movement of a distal portion (30) of an interventional device (3) and temporal information of the periodically moving anatomy (41);
ascertaining (103) by the processor whether the temporal information on the movement of the distal portion (30) of the interventional device (3) is periodic or aperiodic;
gating (104) the temporal information of the periodically moving anatomy (41) by the processor when periodicity is ascertained;
outputting (105) the temporal information of the periodically moving anatomy (41) as received when the temporal information on the movement of the distal portion (30) of the interventional device (3) is ascertained aperiodic, and
outputting gated temporal information of the periodically moving anatomy (41) when the temporal information on the movement of the distal portion (30) of the interventional device (3) is ascertained periodic.
